# EUROPÄISCHE PATENTANMELDUNG

(11) **EP 3 581 560 A1**
(43) Veröffentlichungstag der Anmeldung: **18.12.2019**
(21) Anmeldenummer: 18177699.8
(22) Anmeldetag: 14.06.2018
(51) Int. Cl.: C07C 67/36, C07C 69/92

(54) **VERFAHREN ZUR ALKOXYCARBONYLIERUNG VON DIAZONIUMSALZEN UNTER VERWENDUNG VON BISPHOSPHITEN**

(71) Anmelder: Evonik Operations GmbH, 45128 Essen (DE)
(72) Erfinder: WU, Xiao-Feng, 18059 Rostock (DE); XU, Jianxing, 18059 Rostock (DE); BÖRNER, Armin, 18059 Rostock (DE); FRANKE, Robert, 45772 Marl (DE)
(74) Vertreter: Evonik Patent Association

(57) **Zusammenfassung**

Verfahren zur Alkoxycarbonylierung von Diazoniumsalzen unter Verwendung von Bisphosphiten.

## Beschreibung

Die Erfindung betrifft ein Verfahren zur Alkoxycarbonylierung von Diazoniumsalzen unter Verwendung von Bisphosphiten.

Bei der klassischen Alkoxycarbonylierung wird eine ethylenisch ungesättigte Verbindung (Olefinen) mit Kohlenmonoxid und Alkoholen in Gegenwart eines Metall-Ligand-Komplexes zu den entsprechenden Estern umgesetzt.
Das folgende Schema zeigt die allgemeine Reaktionsgleichung einer klassischen Alkoxycarbonylierung:

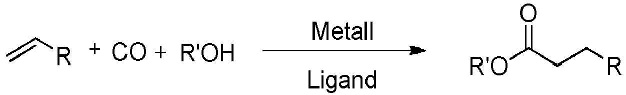

Die vorliegende Erfindung hat die Aufgabe, ein kostengünstiges Alkoxycarbonylierungsverfahren für Diazoniumsalze bereitzustellen, welches zu guten Ausbeuten führt.

Diese Aufgabe wird gelöst durch ein Verfahren nach Anspruch 1.

Verfahren umfassend die Verfahrensschritte:
a) Zugabe eines Diazoniumsalzes;
b) Zugabe einer Verbindung gemäß der Formel (**I**): 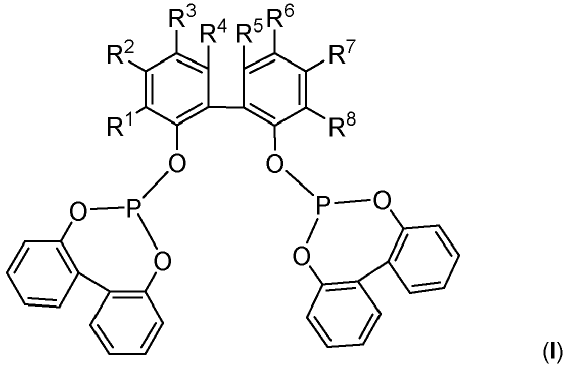 wobei R¹, R², R³, R⁴, R⁵, R⁶, R⁷, R⁸ jeweils unabhängig voneinander ausgewählt sind aus: -H, -(C₁-C₁₂)-Alkyl, -O-(C₁-C₁₂)-Alkyl, -O-(C₆-C₂₀)-Aryl, -(C₆-C₂₀)-Aryl, -(C₃-C₁₂)-Cycloalkyl, Halogen;
c) Zugabe eines Alkohols;
d) Zuführen von CO;
e) Umsetzen des Diazoniumsalzes zu einem Ester;
wobei der Reaktion kein Metall der Gruppen 8, 9, 10 des Periodensystems zugesetzt wird, oder Verbindungen, welche ein solches Metall umfassen.

Periodensystem Gruppe 8: Fe, Ru, Os.
Periodensystem Gruppe 9: Co, Rh, Ir.
Periodensystem Gruppe 10: Ni, Pd, Pt.

Im Rahmen der Erfindung umfasst der Ausdruck -(C₁-C₁₂)-Alkyl geradkettige und verzweigte Alkylgruppen. Vorzugsweise handelt es sich dabei um unsubstituierte geradkettige oder verzweigte -(C₁-C₈)-Alkyl- und ganz bevorzugt -(C₁-C₆)-Alkylgruppen. Beispiele für -(C₁-C₁₂)-Alkylgruppen sind insbesondere Methyl, Ethyl, Propyl, Isopropyl, *n*-Butyl, *iso*-Butyl, *sec*.-Butyl, *tert*.-Butyl, *n*-Pentyl, 2-Pentyl, 2-Methylbutyl-, 3-Methylbutyl-, 1,2-Dimethylpropyl-, 1,1-Dimethylpropyl, 2,2-Dimethylpropyl-, 1-Ethylpropyl-, n-Hexyl-, 2-Hexyl-, 2-Methylpentyl-, 3-Methylpentyl-, 4-Methylpentyl-, 1,1-Dimethylbutyl-, 1,2-Dimethylbutyl-, 2,2-Dimethylbutyl-, 1,3-Dimethylbutyl-, 2,3-Dimethylbutyl-, 3,3-Dimethylbutyl-, 1,1,2-Trimethylpropyl-, 1,2,2-Trimethylpropyl-, 1-Ethylbutyl-, 1-Ethyl-2-methylpropyl-, *n*-Heptyl-, 2-Heptyl-, 3-Heptyl-, 2-Ethylpentyl-, 1-Propylbutyl-, *n*-Octyl-, 2-Ethylhexyl-, 2-Propylheptyl-, Nonyl-, Decyl.

Der Ausdruck -(C₃-C₁₂)-Cycloalkyl umfasst im Sinne der vorliegenden Erfindung mono-, bi- oder tricyclische Kohlenwasserstoffreste mit 3 bis 12, insbesondere 5 bis 12 Kohlenstoffatomen. Dazu zählen Cyclopropyl-, Cyclobutyl-, Cyclopentyl-, Cyclohexyl-, Cycloheptyl-, Cyclooctyl-, Cyclododecyl-, Cyclopentadecyl-, Norbonyl- oder Adamantyl.

Der Ausdruck -(C₆-C₂₀)-Aryl und -(C₆-C₂₀)-Aryl-(C₆-C₂₀)-Aryl- umfasst im Sinne der vorliegenden Erfindung mono- oder polycyclische aromatische Kohlenwasserstoffreste. Diese weisen 6 bis 20 Ringatome, besonders bevorzugt 6 bis 14 Ringatome, insbesondere 6 bis 10 Ringatome, auf. Aryl steht vorzugsweise für -(C₆-C₁₀)-Aryl und -(C₆-C₁₀)-Aryl-(C₆-C₁₀)-Aryl-. Aryl steht insbesondere für Phenyl, Naphthyl, Indenyl, Fluorenyl, Anthracenyl, Phenanthrenyl, Naphthacenyl, Chrysenyl, Pyrenyl, Coronenyl. Insbesondere steht Aryl für Phenyl, Naphthyl und Antracenyl.

Die Verfahrensschritte a), b), c) und d) können in beliebiger Reihenfolge erfolgen. Üblicherweise erfolgt die Zugabe von CO jedoch, nachdem die Reaktionspartner in den Schritten a) bis c) vorgelegt wurden. Die Schritte d) und e) können gleichzeitig oder nacheinander erfolgen. Darüber hinaus kann CO auch in mehreren Schritten zugeführt werden.

In einer Variante des Verfahrens sind R¹, R², R³, R⁴, R⁵, R⁶, R⁷, R⁸ jeweils unabhängig voneinander ausgewählt aus: -H, -(C₁-C₁₂)-Alkyl, -O-(C₁-C₁₂)-Alkyl.

In einer Variante des Verfahrens stehen R², R⁴, R⁵, R⁷ jeweils für -H.

In einer Variante des Verfahrens sind R¹, R³, R⁶, R⁸ jeweils unabhängig voneinander ausgewählt aus: -(C₁-C₁₂)-Alkyl, -O-(C₁-C₁₂)-Alkyl.

In einer Variante des Verfahrens stehen R¹, R⁸ jeweils für -(C₁-C₁₂)-Alkyl.

In einer Variante des Verfahrens stehen R³, R⁶ jeweils für -O-(C₁-C₁₂)-Alkyl.

In einer Variante des Verfahrens weist die Verbindung in Verfahrensschritt b) die Formel (1) auf:

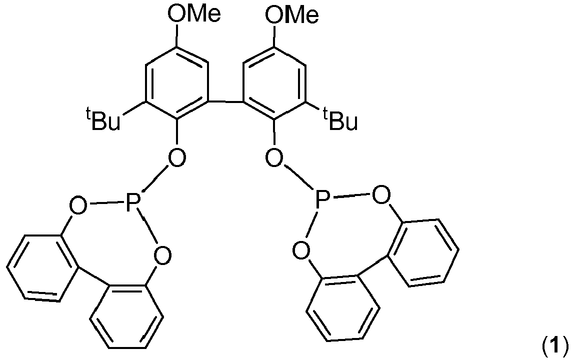

In einer Variante des Verfahrens handelt es sich bei dem Diazoniumsalz um ein Tetrafluoroborat.

In einer Variante des Verfahrens weist das Diazoniumsalz die folgende Formel (**IIa**) auf: 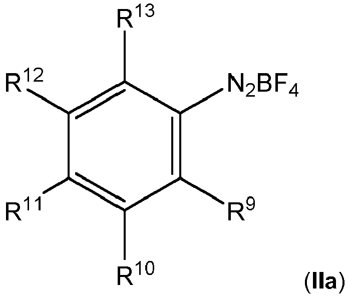 wobei
R⁹, R¹⁰, R¹¹, R¹², R¹³ jeweils unabhängig voneinander ausgewählt sind aus: -H, -(C₁-C₁₂)-Alkyl, -O-(C₁-C₁₂)-Alkyl, -O-(C₆-C₂₀)-Aryl, -(C₆-C₂₀)-Aryl, -(C₃-C₁₂)-Cycloalkyl, Halogen, -NO₂,-CN, -S-(C₁-C₁₂)-Alkyl.

In einer Variante des Verfahrens sind R⁹, R¹⁰, R¹¹, R¹², R¹³ jeweils unabhängig voneinander ausgewählt aus: -H, -(C₁-C₁₂)-Alkyl, -O-(C₁-C₁₂)-Alkyl.

In einer Variante des Verfahrens weist das Diazoniumsalz die folgende Formel (**IIb**) auf: 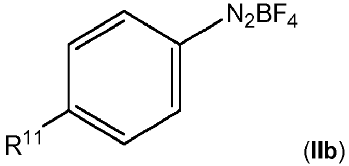 wobei
R¹¹ für einen Rest steht ausgewählt aus: -H, -(C₁-C₁₂)-Alkyl, -O-(C₁-C₁₂)-Alkyl, -O-(C₆-C₂₀)-Aryl, -(C₆-C₂₀)-Aryl, -(C₃-C₁₂)-Cycloalkyl, Halogen, -NO₂, -CN, -S-(C₁-C₁₂)-Alkyl.

In einer Variante des Verfahrens ist R¹¹ ausgewählt aus: -H, -(C₁-C₁₂)-Alkyl, -O-(C₁-C₁₂)-Alkyl.

In einer Variante des Verfahrens erfolgt das Umsetzen im Verfahrensschritt e) bei einer Temperatur im Bereich von 10 °C bis 40 °C.

In einer Variante des Verfahrens erfolgt das Umsetzen im Verfahrensschritt e) bei einer Temperatur im Bereich von 20 °C bis 30 °C.

In einer Variante des Verfahrens erfolgt das Umsetzen im Verfahrensschritt e) bei Raumtemperatur.

In einer Variante des Verfahrens erfolgt das Zuführen von CO im Verfahrensschritt d) so, dass die Reaktion bei einem CO-Druck im Bereich von 30 bar bis 70 bar verläuft.

In einer Variante des Verfahrens erfolgt das Zuführen von CO im Verfahrensschritt d) so, dass die Reaktion bei einem CO-Druck im Bereich von 40 bar bis 60 bar verläuft.

In einer Variante des Verfahrens ist der Alkohol in Verfahrensschritt c) ausgewählt aus: Methanol, Ethanol, 1-Propanol, 1-Butanol, 1-Pentanol, 1-Hexanol, 2-Propanol, *tert*-Butanol, 3-Pentanol, Cyclohexanol, Phenol, oder Mischungen davon.

In einer Variante des Verfahrens ist der Alkohol in Verfahrensschritt c) Methanol.

Im Folgenden wird die Erfindung anhand von Ausführungsbeispielen näher erläutert.

### Allgemeine Versuchsvorschrift für Autoklavenversuche in Glasvials:

Es wird ein 300ml Parrreaktor verwendet. Diesem angepasst ist ein selbstgefertigter Aluminiumblock entsprechender Dimension welcher zu Heizen mittels handelsüblicher Magnetrührer z.B. der Firma Heidolph geeignet ist. Für das Innere des Autoklaven wurde eine runde Metallplatte der Stärke von ca. 1,5 cm angefertigt die 6 Bohrungen enthält, die dem Außendurchmesser der Glasvials entsprechen. Diesen Glasvials angepasst werden sie mit kleinen Magnetrührern bestückt. Diese Glasvials werden mit Schraubkappen und geeigneten Septen versehen und mit einer in der Glasbläserei angefertigten Spezialapparatur unter Argon mit den entsprechenden Reaktanden, Lösungsmittel und Katalysatoren und Additiven befüllt. Hierzu werden 6 Gefäße gleichzeitig befüllt, dies ermöglicht die Durchführung von 6 Reaktionen bei gleicher Temperatur und gleichem Druck in einem Experiment. Dann werden diese Glasgefäße mit Schraubkappen und Septen geschlossen und es wird jeweils ein kleine Spritzenkanüle geeigneter Größe durch die Septen gestochen. Dies ermöglicht später in der Reaktion den Gasaustausch. Diese Vials werden nun in der Metallplatte platziert und diese wird unter Argon in den Autoklaven überführt. Der Autoklav wird mit CO gespült und bei Raumtemperatur mit dem vorgesehenen CO-Druck befüllt. Dann wird mittels dem Magnetrührer unter Magnetrührung auf Reaktionstemperatur gebracht und die Reaktion die entsprechende Zeit durchgeführt. Anschließend wird der Autoklav mit Stickstoff gespült.

### Verbindungen

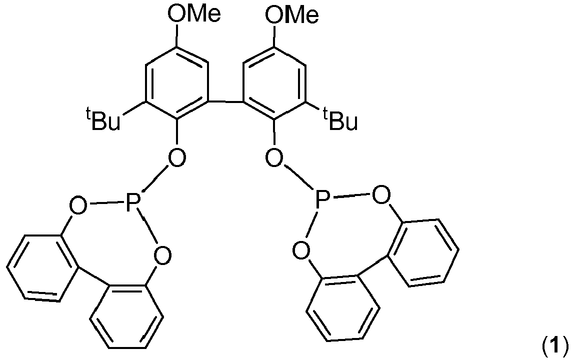

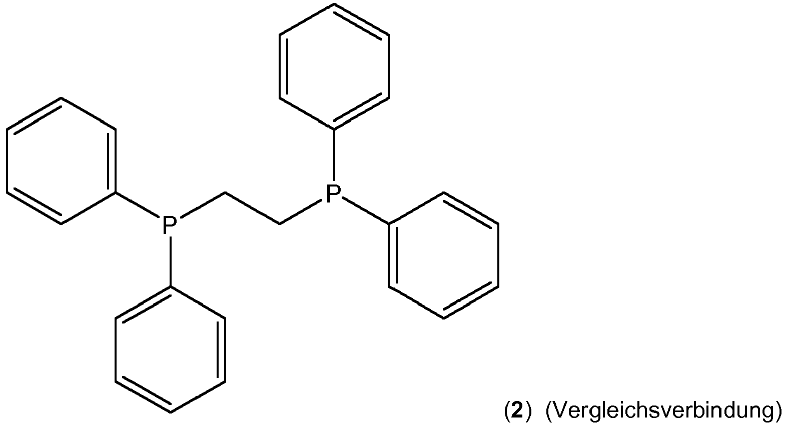

### Reaktionsschema

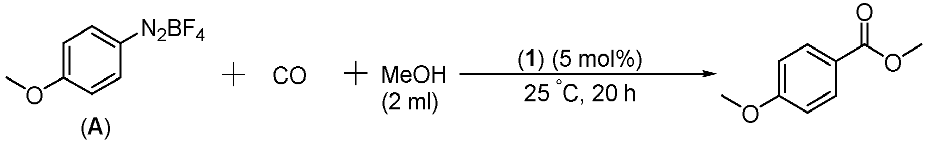

### Methoxycarbonylierung

Ein 4 mL-Fläschchen wurde mit Diazoniumsalz (**A**) (0,4 mmol) und Verbindung (**1**) (5 mol%) befüllt und ein Magnetrührstab hinzugegeben. Anschließend wurde das Fläschchen mit Argon befüllt und MeOH (2 ml) hinzugegeben. Das Fläschchen wurde auf einen Probenhalter gesetzt, der wiederum unter Argon-Atmosphäre in einen 300 ml-Parr-Autoklaven eingesetzt wurde. Nach dreimaligem Spülen des Autoklaven mit CO wurde der CO-Druck auf 50 bar eingestellt. Die Reaktion lief bei 25 °C 20 Stunden.

Der oben beschriebene Versuch wurde mit der Verbindung (**2**) wiederholt. Die anderen Parameter wurden beibehalten. Die Ergebnisse der Versuchsreihe sind in der nachfolgenden Tabelle zusammengestellt:

**Tabelle:**

| Verbindung (**X**) | CO-Druck [bar] | Ausbeute [%] |
|---|---|---|
| **1*** | 50 | 40 |
| **2** | 50 | 19 |

| | | |
|---|---|---|
| * erfindungsgemäßes Verfahren | | |

Wie die oben gelisteten Ergebnisse zeigen, wird die Aufgabe durch ein erfindungsgemäßes Verfahren gelöst.

## Patentansprüche

1. Verfahren umfassend die Verfahrensschritte:
a) Zugabe eines Diazoniumsalzes;
b) Zugabe einer Verbindung gemäß der Formel (**I**): 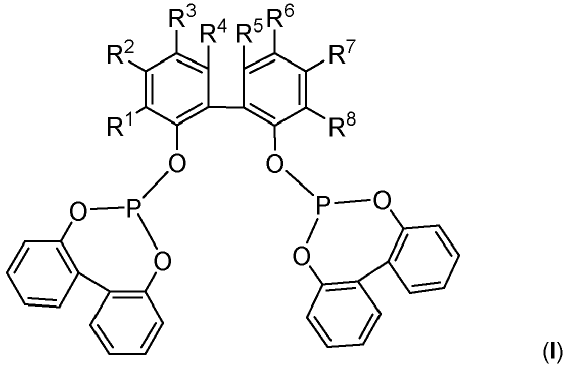 wobei
R¹, R², R³, R⁴, R⁵, R⁶, R⁷, R⁸ jeweils unabhängig voneinander ausgewählt sind aus: -H, -(C₁-C₁₂)-Alkyl, -O-(C₁-C₁₂)-Alkyl, -O-(C₆-C₂₀)-Aryl, -(C₆-C₂₀)-Aryl, -(C₃-C₁₂)-Cycloalkyl, Halogen;
c) Zugabe eines Alkohols;
d) Zuführen von CO;
e) Umsetzen des Diazoniumsalzes zu einem Ester;
wobei der Reaktion kein Metall der Gruppen 8, 9, 10 des Periodensystems zugesetzt wird, oder Verbindungen, welche ein solches Metall umfassen.

2. Verfahren nach Anspruch 1,
wobei R¹, R², R³, R⁴, R⁵, R⁶, R⁷, R⁸ jeweils unabhängig voneinander ausgewählt sind aus: -H, -(C₁-C₁₂)-Alkyl, -O-(C₁-C₁₂)-Alkyl.

3. Verfahren nach Anspruch 1 oder 2,
wobei R², R⁴, R⁵, R⁷ jeweils für -H stehen.

4. Verfahren nach einem der Ansprüche 1 bis 3,
wobei R¹, R³, R⁶, R⁸ jeweils unabhängig voneinander ausgewählt sind aus: -(C₁-C₁₂)-Alkyl,-O-(C₁-C₁₂)-Alkyl.

5. Verfahren nach einem der Ansprüche 1 bis 4,
wobei die Verbindung in Verfahrensschritt b) die Formel (1) aufweist:

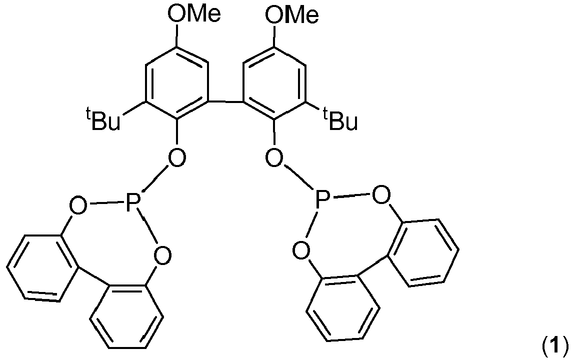

6. Verfahren nach einem der Ansprüche 1 bis 5,
wobei es sich bei dem Diazoniumsalz um ein Tetrafluoroborat handelt.

7. Verfahren nach einem der Ansprüche 1 bis 6,
wobei das Diazoniumsalz die folgende Formel (**IIa**) aufweist: 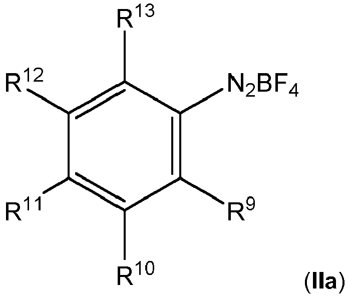 wobei
R⁹, R¹⁰, R¹¹, R¹², R¹³ jeweils unabhängig voneinander ausgewählt sind aus: -H, -(C₁-C₁₂)-Alkyl, -O-(C₁-C₁₂)-Alkyl, -O-(C₆-C₂₀)-Aryl, -(C₆-C₂₀)-Aryl, -(C₃-C₁₂)-Cycloalkyl, Halogen, -NO₂,-CN, -S-(C₁-C₁₂)-Alkyl.

8. Verfahren nach Anspruch 7,
wobei R⁹, R¹⁰, R¹¹, R¹², R¹³ jeweils unabhängig voneinander ausgewählt sind aus: -H, -(C₁-C₁₂)-Alkyl, -O-(C₁-C₁₂)-Alkyl.

9. Verfahren nach einem der Ansprüche 1 bis 6,
wobei das Diazoniumsalz die folgende Formel (**IIb**) aufweist: 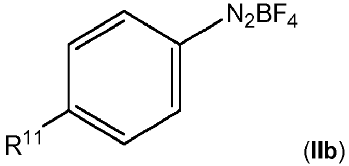 wobei
R¹¹ für einen Rest steht ausgewählt aus: -H, -(C₁-C₁₂)-Alkyl, -O-(C₁-C₁₂)-Alkyl, -O-(C₆-C₂₀)-Aryl, -(C₆-C₂₀)-Aryl, -(C₃-C₁₂)-Cycloalkyl, Halogen, -NO₂, -CN, -S-(C₁-C₁₂)-Alkyl.

10. Verfahren nach einem der Ansprüche 1 bis 9,
wobei das Umsetzen im Verfahrensschritt e) bei einer Temperatur im Bereich von 10 °C bis 40 °C erfolgt.

11. Verfahren nach einem der Ansprüche 1 bis 10,
wobei das Zuführen von CO im Verfahrensschritt d) so erfolgt, dass die Reaktion bei einem CO-Druck im Bereich von 30 bar bis 70 bar verläuft.

12. Verfahren nach einem der Ansprüche 1 bis 11,
wobei der Alkohol in Verfahrensschritt c) ausgewählt ist aus: Methanol, Ethanol, 1-Propanol, 1-Butanol, 1-Pentanol, 1-Hexanol, 2-Propanol, *tert*-Butanol, 3-Pentanol, Cyclohexanol, Phenol, oder Mischungen davon.

## Geänderte Patentansprüche

### Geänderte Patentansprüche gemäss Regel 137(2) EPÜ.

1. Verfahren umfassend die Verfahrensschritte:
a) Zugabe eines Diazoniumsalzes,
wobei das Diazoniumsalz die folgende Formel (**IIa**) aufweist: 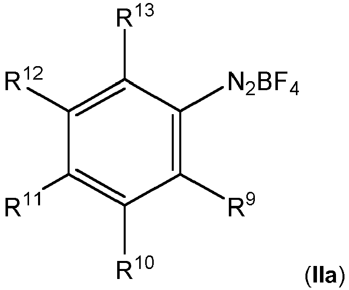 wobei
R⁹, R¹⁰, R¹¹, R¹², R¹³ jeweils unabhängig voneinander ausgewählt sind aus: -H, -(C₁-C₁₂)-Alkyl, -O-(C₁-C₁₂)-Alkyl, -O-(C₆-C₂₀)-Aryl, -(C₆-C₂₀)-Aryl, -(C₃-C₁₂)-Cycloalkyl, Halogen, -NO₂, - CN, -S-(C₁-C₁₂)-Alkyl;
b) Zugabe einer Verbindung gemäß der Formel (**I**): 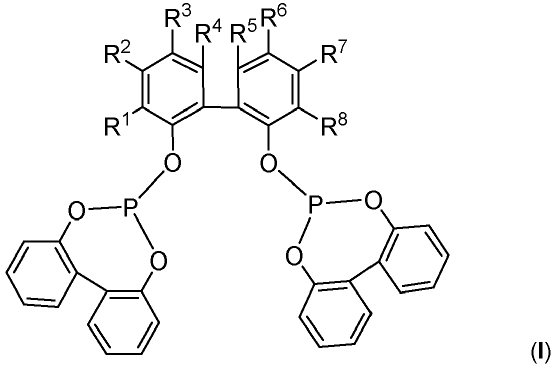 wobei
R¹, R², R³, R⁴, R⁵, R⁶, R⁷, R⁸ jeweils unabhängig voneinander ausgewählt sind aus: -H, -(C₁-C₁₂)-Alkyl, -O-(C₁-C₁₂)-Alkyl, -O-(C₆-C₂₀)-Aryl, -(C₆-C₂₀)-Aryl, -(C₃-C₁₂)-Cycloalkyl, Halogen;
c) Zugabe eines Alkohols;
d) Zuführen von CO;
e) Umsetzen des Diazoniumsalzes zu einem Ester;
wobei der Reaktion kein Metall der Gruppen 8, 9, 10 des Periodensystems zugesetzt wird, oder Verbindungen, welche ein solches Metall umfassen.

2. Verfahren nach Anspruch 1,
wobei R¹, R², R³, R⁴, R⁵, R⁶, R⁷, R⁸ jeweils unabhängig voneinander ausgewählt sind aus: -H, -(C₁-C₁₂)-Alkyl, -O-(C₁-C₁₂)-Alkyl.

3. Verfahren nach Anspruch 1 oder 2,
wobei R², R⁴, R⁵, R⁷ jeweils für -H stehen.

4. Verfahren nach einem der Ansprüche 1 bis 3,
wobei R¹, R³, R⁶, R⁸ jeweils unabhängig voneinander ausgewählt sind aus: -(C₁-C₁₂)-Alkyl, - O-(C₁-C₁₂)-Alkyl.

5. Verfahren nach einem der Ansprüche 1 bis 4,
wobei die Verbindung in Verfahrensschritt b) die Formel (**1**) aufweist:

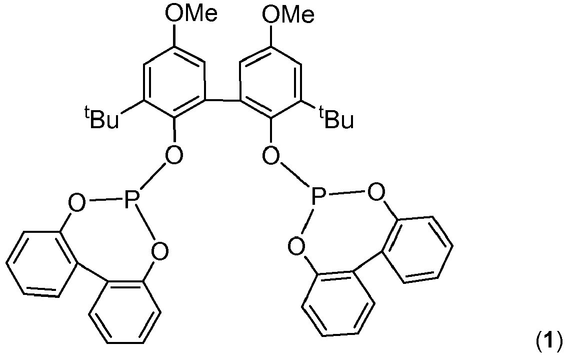

6. Verfahren nach Anspruch 1,
wobei R⁹, R¹⁰, R¹¹, R¹², R¹³ jeweils unabhängig voneinander ausgewählt sind aus: -H, -(C₁-C₁₂)-Alkyl, -O-(C₁-C₁₂)-Alkyl.

7. Verfahren nach einem der Ansprüche 1 bis 6,
wobei das Diazoniumsalz die folgende Formel (**IIb**) aufweist: 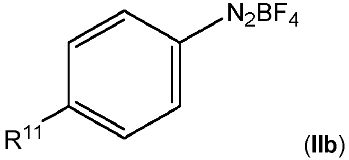 wobei
R¹¹ für einen Rest steht ausgewählt aus: -H, -(C₁-C₁₂)-Alkyl, -O-(C₁-C₁₂)-Alkyl, -O-(C₆-C₂₀)-Aryl, -(C₆-C₂₀)-Aryl, -(C₃-C₁₂)-Cycloalkyl, Halogen, -NO₂, -CN, -S-(C₁-C₁₂)-Alkyl.

8. Verfahren nach einem der Ansprüche 1 bis 7,
wobei das Umsetzen im Verfahrensschritt e) bei einer Temperatur im Bereich von 10 °C bis 40 °C erfolgt.

9. Verfahren nach einem der Ansprüche 1 bis 8,
wobei das Zuführen von CO im Verfahrensschritt d) so erfolgt, dass die Reaktion bei einem CO-Druck im Bereich von 30 bar bis 70 bar verläuft.

10. Verfahren nach einem der Ansprüche 1 bis 9,
wobei der Alkohol in Verfahrensschritt c) ausgewählt ist aus: Methanol, Ethanol, 1-Propanol, 1-Butanol, 1-Pentanol, 1-Hexanol, 2-Propanol, *tert*-Butanol, 3-Pentanol, Cyclohexanol, Phenol, oder Mischungen davon.
